# FASCICULE DE BREVET EUROPEEN

(11) **EP 2 909 310 B1**
(45) Date de publication et mention de la délivrance du brevet: **27.02.2019**
(21) Numéro de dépôt: 13785521.9
(22) Date de dépôt: 08.10.2013
(51) Int. Cl.: A21D 8/04, C12N 1/18, C12N 9/04, C12N 9/12, C12N 9/90, C12N 9/92, C12N 15/81, C12N 1/22, C12R 1/865, C12P 7/10, C12N 15/01

(54) **SOUCHES DE LEVURES POUR LA PRODUCTION DE BIOMASSE SUR UN SUBSTRAT COMPRENANT UN SUCRE EN C5**
HEFESTÄMME ZUR HERSTELLUNG VON BIOMASSE AUF EINEM SUBSTRAT MIT EINEM C5-ZUCKER
YEAST STRAINS FOR THE PRODUCTION OF BIOMASS ON A SUBSTRATE COMPRISING A C5 SUGAR

(30) Priorité: 16.10.2012 FR 1259836
(43) Date de publication de la demande: 26.08.2015
(73) Titulaire: Lesaffre et Compagnie, 75001 Paris (FR)
(72) Inventeur: DESFOUGERES, Thomas, 59960 Neuville en Ferrain (FR); PIGNEDE, Georges, 59700 Marcq-en-Baroeul (FR)
(74) Mandataire: Cabinet Plasseraud
(86) Numéro de dépôt international: PCT/FR2013/052391
(87) Numéro de publication internationale: WO 2014/060678

(56) Documents cités:
- WO-A1-2011/003893
- WO-A1-2011/128552
- WO-A1-2011/131667
- KARHUMAA KAISA ET AL: "Investigation of limiting metabolic steps in the utilization of xylose by recombinant Saccharomyces cerevisiae using metabolic engineering", YEAST, JOHN WILEY & SONS LTD, GB, vol. 22, no. 5, avril 2005 (2005-04), pages 359-368, XP002347259, ISSN: 0749-503X, DOI: 10.1002/YEA.1216
- PAUL V ATTFIELD ET AL: "Use of population genetics to derive nonrecombinant Saccharomyces cerevisiae strains that grow using xylose as a sole carbon source", FEMS YEAST RESEARCH, WILEY-BLACKWELL PUBLISHING LTD, GB, NL , vol. 6, no. 6 septembre 2006 (2006-09), pages 862-868, XP002672039, ISSN: 1567-1356, DOI: 10.1111/J.1567-1364.2006.00098.X Extrait de l'Internet: URL:http://onlinelibrary.wiley.com/doi/10. 1111/j.1567-1364.2006.00098.x/abstract [extrait le 2006-06-05]
- BAERBEL HAHN-HAEGERDAL ET AL: "Metabolic Engineering of Saccharomyces cerevisiae for Xylose Utilization", ADVANCES IN BIOCHEMICAL ENGINEERING, BIOTECHNOLOGY, SPRINGER, BERLIN, DE, vol. 73, janvier 2001 (2001-01), pages 53-84, XP002980022, ISSN: 0724-6145, DOI: 10.1007/3-540-45300-8_4

## Description

### Domaine technique:

La présente invention se rapporte au domaine de la production de biomasse, notamment à la production de levures. Elle concerne particulièrement la production de levures sur un substrat comprenant au moins un sucre en C5 et plus particulièrement à la production de levures sur un substrat issu de l'hydrolyse de matériaux lignocellulosiques. Elle concerne également l'utilisation des levures produites dans des applications pour l'industrie de la levure telles que la panification, la production de biomasse, la production d'arômes, la production de métabolites secondaires, la production de protéine, la production d'ARN, la production d'éthanol, la brasserie ou la production d'extrait de levure.

### Arrière-plan technologique et problème à résoudre:

Pour leur multiplication par un processus de respiration aérobie, les levures ont besoin de :
- composés carbonés source de carbone et d'énergie,
- composés azotés réduits sous forme d'ammonium, quelques levures peuvent cependant utiliser des composés oxydés (comme les nitrates) ou organiques pour la synthèse de protéines et d'acides nucléiques,
- éléments minéraux variés, vitamines et facteurs de croissance qui varient selon les levures.

Si la levure est produite sur un milieu de composition définie, à base, par exemple, de sirops de glucose, ou de fructose comme substrat carboné et de sels d'ammonium et de phosphore comme sources d'azote et de phosphate, le milieu de culture devra être complémenté par un apport de sels minéraux, de vitamines et d'oligoéléments, ce qui peut rendre la production complexe et onéreuse.

Les mélasses de sucrerie de betterave ou de canne sont constituées de sucres (glucose et fructose), de composés organiques et de composés minéraux. C'est pourquoi ces mélasses sont des substrats de choix sur les plans économique et technique et sont, à ce jour, la principale matière première utilisée pour la production industrielle de levures.

Cependant, la raréfaction des mélasses de canne et de betterave, en particulier liée à leur utilisation massive pour la production de bioéthanol, met en péril ce type de production. Il est de ce fait nécessaire de trouver de nouveaux substrats carbonés en substitution du saccharose fourni par les mélasses.

Or, la production industrielle de bioéthanol, notamment le bioéthanol de deuxième génération, a mis en lumière la disponibilité d'autres sources de substrats de fermentation. En effet, l'industrie du bioéthanol de deuxième génération utilise des matériaux lignocellulosiques dérivés de plantes. Ces matériaux qui représentent la vaste majorité des végétaux sont constitués de cellulose, d'hémicellulose et de lignine. Après hydrolyse, les matériaux lignocellulosiques conduisent à un mélange de sucres en C6 (6 atomes de carbone) et en C5 (5 atomes de carbone).

Toutes les levures sont capables de métaboliser les sucres en C6, en revanche et de manière générale, les sucres en C5 sont faiblement fermentescibles, voire non fermentescibles par les levures du genre *Saccharomyces*, notamment *Saccharomyces cerevisiae* (Sc).

La littérature récente relative à la production de bioéthanol de deuxième génération, décrit des levures de Sc modifiées pour les rendre aptes à fermenter les sucres en C5. Parmi ces documents, on peut citer à titre indicatif, WO 2011/128552 et WO 2012/72793 au nom de la demanderesse.

WO 2011/128552 décrit une souche de levure obtenue par un procédé de modification génétique comprenant l'introduction dans le génome de la souche d'un gène codant pour une Xylose réductase (XR) et d'un gène codant pour une Xylose déshydrogénase (XDH).

WO 2012/72793 décrit une souche de levure obtenue par un procédé comprenant l'introduction dans le génome de la souche d'une copie d'un gène exogène codant pour une xylose isomérase, ledit gène étant de préférence issu de *Clostridium phytofermentans* et d'au moins une copie d'un gène codant pour une xylitol déshydrogénase, ledit gène étant issu de *Pichia stipitis.*

Le l'article de Attfield et al (« Use ofpopulation genetics to derive nonrecombinant Saccharomyces cerevisiae strains that grow using xylose as a sole carbon source», Fems Yeast research, Wiley-Blackwell Publishing LTD, GB, NL, vol. 6, no. 6, septembre 2006, pages 862-868) divulgue la production de souches non-recombinantes aptes à se multiplier en conditions d'aérobies en ayant du xylose comme seule source de carbone.

Bien que les souches décrites dans ces documents soient capables de se multiplier sur un substrat comprenant un sucre en C5, les inventeurs de la présente ont constaté que la vitesse de multiplication sur un substrat constitué ou comprenant un sucre en C5 reste faible et en tout cas est incompatible avec une production industrielle de levures.

De plus, les inventeurs ont constaté que lors de leur utilisation industrielle finale, par exemple en tant que levures de fermentation panaire, ou levures destinées à la production d'extrait de levures, etc, les levures ainsi obtenues présentaient une efficacité réduite par rapport à l'efficacité des levures obtenues à partir des souches d'origine non modifiées.

Il existe donc un réel besoin en de nouvelles souches pour la production de levures qui permettent de remplacer totalement ou partiellement les sucres en C6 en tant que substrat carboné, avec des rendements de production de levure compatibles avec une exploitation économique et industrielle. En outre, la culture de telles souches devraient conduire à des levures présentant une efficacité dans leur utilisation industrielle finale tout à fait intéressante et satisfaisante.

### Résumé de l'invention

La présente invention a pour objet de nouvelles souches de levures de Sc aptes à se multiplier sur un substrat comprenant au moins un sucre en C5 avec une vitesse et un taux de multiplication compatibles avec la production industrielle de levure.

Elle porte également sur des souches nouvelles dont la culture permet d'obtenir des levures présentant une efficacité applicative, c'est-à-dire une efficacité dans leur utilisation industrielle finale satisfaisante, c'est-à-dire au moins égale à 80%, de préférence 95% et plus préférentiellement encore 105% de l'efficacité applicative de référence.

Un autre objet de l'invention est l'utilisation des levures obtenues par le procédé de l'invention et/ou des nouvelles levures dans une application industrielle choisie parmi la panification, la production de biomasse, la production d'arômes, la production de métabolites secondaires, la production de protéine, la production d'ARN, la production d'éthanol, la brasserie, l'oenologie ou la production d'extrait de levure.

### Brève description des figures:

La figure 1 est le résultat d'une analyse PCR de l'exemple 1. Les valeurs indiquées sur la partie gauche du gel correspondent à la taille des fragments d'Adn du marqueur de taille, exprimées en paires de base (piste 1). Les pistes 2 à 5 correspondent respectivement à X10S1, X10S2, Témoin Mat a et Témoin Mat alpha.
La figure 2 est un graphe représentant l'évolution de la turbidité du milieu exprimée en Log 10(DO _{lambda = 600 nm}) en fonction du temps de culture (exprimé en heures) à 30°C et à 150 rpm dans du milieu CP.
La figure 3 est un graphe représentant la turbidité du milieu exprimée en DO à une longueur d'onde de 600 nm, en fonction du temps de culture, exprimé en heures, à 30 °C et à 150 rpm dans un milieu YFX.
La figure 4 est un graphe représentant le dégagement gazeux exprimé en Ml par minute sur pâte normale au cours du temps exprimé en minutes.

### Description détaillée de l'invention:

La présente invention a pour objet une souche de levure apte à se multiplier en condition aérobie sur un substrat comprenant au moins un sucre en C5 caractérisée en ce qu'elle satisfait un test de croissance sur xylose ou qu'elle présente une vitesse de multiplication maximale telle qu'elle satisfait un test de croissance sur milieu CP.

### Test de croissance sur xylose:

Ce test consiste à suivre au cours du temps par spectrophotométrie à une longueur d'onde de 600 nm l'évolution de la turbidité d'une solution contenant 50mL d'un milieu de culture YFX inoculée par 10⁸ cellules de souche à tester à 30°C sous agitation constante à 150 rpm. Le test est satisfait lorsque la Densité Optique (DO) à 24H est supérieure à 2, et est d'au moins 3, de préférence d'au moins 10 à 48H.

Le milieu YFX est tel que défini ci-dessous:

| YFX | g/kg | mL |
|---|---|---|
| Eau distillée qsp | 1000 | |
| Xylose | 70 | |
| EXL type J | 5 | |
| DAP | 4,7 | |
| Acide citrique | 11,4 | |
| Citrate trisodique | 13,5 | |
| ZnSO4(10,6 g/L) | | 2 |
| MgSO4 7H2O (400 g/L) | | 2,5 |
| Thiamine Vit B1(18,24 g/L) | | 1 |
| Pyrodixine Vit B6 (5,28 g/L) | | 1 |
| Biotine (1,76 g/L) + KOH | | 1 |
| Panthoténate (3,8 g/L) | | 1 |
| Acide nicotinique (4 g/L) | | 4 |
| Mésoinositol (50 g/L) | | 1 |
| Riboflavine (1 g/L) | | 1 |
| Para aminobenzoate (1,2 g/L) | | 1 |
| Tween 80 | | 1 |

### Test de croissance sur milieu CP :

Ce test consiste à suivre au cours du temps par spectrophotométrie à une longueur d'onde de 600 nm l'évolution de la turbidité d'une solution contenant 50 mL d'un milieu de culture CP qui contient 10 g/kg d'EXL type J100 (Biospringer) et 10 g/kg de bactopeptone ainsi que 100 g/kg de saccharose, ledit milieu étant inoculé par 10⁸ cellules à tester à 30°C sous agitation constante à 150 rpm. Le test est satisfait lorsqu'entre 3,5 et 7,5 heures l'évolution de la DO est exponentielle. L'équation qui représente alors l'évolution de la DO est de la forme DO_{tf} = DOₜᵢ e^{n(tf-ti)} avec n compris entre 0,35 et 0,9 de préférence entre 0,45 et 0,80 et préférentiellement entre 0,65 et 0,75.

Selon un mode de réalisation avantageux, les souches selon l'invention satisfont à la fois le test de croissance sur xylose et le test de croissance sur milieu CP.

Selon l'invention, la souche de levure est choisie dans le genre Saccharomyces et de préférence *Saccharomyces cerevisiae.*

Par sucre en C5 selon l'invention, on entend un ose comprenant 5 atomes de carbone ou pentose. Le sucre en C5 est choisi dans le groupe comprenant l'arabinose, le xylose, et leurs mélanges. De préférence, le sucre en C5 est le xylose.

Selon l'invention, le substrat comprenant au moins un sucre en C5 comprend aussi au moins un sucre en C6, c'est-à-dire un ose comprenant 6 atomes de carbone ou hexose. Le sucre en C6 est choisi dans le groupe comprenant le glucose, le fructose, le galactose, le mannose ou issu de la dégradation de diholoside comme le saccharose, le maltose le tréhalose, l'isomaltose et leurs mélanges.

De préférence le substrat de l'invention comprend au moins un sucre en C5 et au moins un sucre en C6 dans un rapport C5/C6 compris entre 0,1 et 2.

Les levures obtenues par la multiplication des souches selon l'invention présentent des performances utiles pour les applications industrielles comme la panification, la production de biomasse, la production d'arômes, la production de métabolites secondaires, la production de protéine, la production d'éthanol, la brasserie, l'oenologie ou la production d'extrait de levure. En d'autres termes, les levures obtenues par culture des souches selon l'invention sont efficaces dans l'utilisation industrielle finale à laquelle elles sont destinées. Cette efficacité est appelée dans la présente demande de brevet "efficacité applicative".

Pour toutes les utilisations industrielles envisagées, il est nécessaire que les levures obtenues par culture des souches de l'invention ne soient pas détériorées au séchage, c'est-à-dire que moins de 20% des levures soient inactivées après un séchage.

Selon un mode de réalisation particulier, les souches selon l'invention sont telles qu'elles satisfont le test de séchage suivant:
La biomasse obtenue après la culture est séparée du milieu par séparation centrifuge en utilisant éventuellement des étapes de lavage, pour obtenir une suspension concentrée de levure d'extrait sec 18-22% (« crème de levure »).

Cette suspension est soumise à une étape de déshydratation par filtration pour obtenir une masse pâteuse (« levure pressée ») à 28-34% d'extrait sec.

Cette masse subit ensuite un traitement de boudinage (extrusion) pour obtenir de fins boudins (vermicelles) de 0,2 à 2 mm de diamètre qui sont séchés par un courant d'air chaud dans un séchoir à lit d'air fluidisé.

Les conditions de séchage (barème de température) sont rigoureusement contrôlées pour que la température du produit soit toujours inférieure à 50°C. La levure sèche finale qui se présente sous la forme de fins vermicelles a un extrait sec d'au moins 95%.
Pour améliorer la viabilité des cellules de levure sèche, on peut ajouter à la levure (suspension ou levure pressée) des additifs à effet protecteur comme des émulsifiants à hauteur de 0,2 à 2%/poids sec de levure et parmi lesquels le monostéarate de sorbitane (MSS) est un additif standard couramment utilisé.

Les souches satisfont le test si le nombre de levures vivantes après séchage est au moins égal à 80% du nombre de levures vivantes avant séchage.

Selon un autre mode de réalisation, la souche selon l'invention présente une efficacité applicative EA au moins égale à 80%, de préférence au moins égale à 95% et plus préférentiellement encore au moins égale à 105% de l'efficacité applicative de référence. Au sens de la présente invention, on entend par une efficacité applicative des souches, une efficacité satisfaisante des levures obtenues par leur culture dans l'utilisation industrielle finale desdites levures, c'est-à-dire une efficacité applicative EA au moins égale à 90% de l'efficacité applicative d'une levure obtenue par culture d'une souche de référence pour cette utilisation. Une souche de référence pour une utilisation donnée est une souche classiquement mise en oeuvre pour cette utilisation. Ainsi, l'efficacité applicative pour une souche dont la culture est destinée à l'industrie panaire sera la force fermentative, et l'efficacité applicative de référence sera celle de la souche déposée le 12 février 2003 à la Collection Nationale des Cultures de Microorganismes (CNCM, Institut Pasteur, 28 rue du Docteur Roux, 75724 Paris cedex 15) sous le N° I-2970 et sera de 120 mL de CO₂ dégagée en 2 heures à partir de 20 g de farine. La méthode de détermination est la méthode dite du Risographe réalisée à l'aide du fermentomètre Burrows et Harris (Journal of Institute of Brewing, vol LXV, N°1, Janvier-Février 25, 1959).

La production de biomasse est réalisée suivant un procédé dit « procédé Fed batch aérobie » désigné aussi par « culture en mode semi-continu » ou « culture semi-continue » ou « mode semi-continu », désigne ici une culture dans un fermenteur (ou réacteur) qui est alimenté progressivement par le milieu de culture, mais dont aucun volume de milieu n'est soutiré. Dans un tel procédé, le volume de culture est variable (et généralement croissant) dans le fermenteur, le débit d'alimentation pouvant être constant ou variable.

Le procédé « Fed Batch » est généralement réalisé dans les conditions décrites dans le livre de référence « Yeast Technology », Chapitre 6, 2ème édition, 1991, G. Reed et T.W. Nagodawithana, publié par Van Nostrand Reinhold, ISBN 0-442-31892-8.

Pour une souche dont la culture est destinée à la production de biomasse, l'efficacité applicative sera le rendement de production et l'efficacité applicative de référence sera celle de la souche déposée le 26 février 1981 à la NCYC (National Collection of Yeast Cultures, Institute of Food Research, Norwich Research Park, Colney, Norwich, United Kingdom, NR4 7UA) sous le N° 995 et sera de 80% de rendement par rapport au sucre C6 mis en oeuvre, soit 80 g de levure par g de mélasse coulée. Le test sera réalisé par mesure de la quantité de matière sèche produite.

Pour une souche dont la culture est destinée à la production d'alcool, l'efficacité applicative sera le rendement de production d'éthanol, et l'efficacité applicative de référence sera celle de la souche déposée le 4 septembre 2008 à la Collection Nationale des Cultures de Microorganismes (CNCM, Institut Pasteur, 28 rue du Docteur Roux, 75724 Paris cedex 15) sous le N° I-4071 et sera de 0,40 g d'éthanol produit par g de sucre C6 mis en oeuvre. Les quantités d'éthanol et de sucre C6 sont mesurées par HPLC ou dosages enzymatiques.

Pour une souche dont la culture est destinée à la production d'extrait de levure, l'efficacité applicative sera la détermination de la teneur en azote de l'extrait de levure obtenu, et l'efficacité applicative de référence sera celle de l'extrait de levure commercialisé par la société Bio Springer sous la référence *Springer 0203*/*0-MG-L* et sera de 10% d'azote par rapport à la matière sèche. Le dosage d'azote se fait par la méthode de combustion.

Selon un mode de réalisation particulier, la souche présente une efficacité applicative EA au moins égale à 80%, de préférence au moins égale à 95% et plus préférentiellement encore au moins égale à 105% de l'efficacité applicative de référence, l'efficacité applicative étant choisie dans le groupe comprenant la force fermentative, le rendement de production de biomasse, d'arôme, de métabolite secondaire, de protéine, d'éthanol.

De façon avantageuse la souche selon l'invention satisfait au test de séchage et présente une efficacité applicative EA au moins égale à 80% de l'efficacité applicative de référence.

L'invention porte également sur les nouvelles souches suivantes :
- souche de levure déposée le 23 août 2012 à la Collection Nationale des Cultures de Microorganismes (CNCM, Institut Pasteur, 28 rue du Docteur Roux, 75724 Paris cedex 15) le sous le N° I-4670
- souche de levure déposée le 23 août 2012 à la Collection Nationale des Cultures de Microorganismes (CNCM, Institut Pasteur, 28 rue du Docteur Roux, 75724 Paris cedex 15) le sous le N° I-4671.

Ces souches sont des souches de levure panaire qui satisfont chacune des tests de croissance sur xylose et de croissance sur milieu CP et qui présentent une force fermentative respectivement d'au moins 100 mL de CO₂ dégagée en 2 heures à partir de 20 g de farine. Ces souches satisfont également le test de séchage.

Un procédé d'obtention d'une souche de levure telle que définie précédemment est également décrit.

Selon une première variante, le procédé comprend une étape de modification génétique d'une souche B présentant une efficacité applicative de référence pour la rendre apte à métaboliser un sucre en C5.

Selon une deuxième variante, le procédé comprend le croisement d'une souche A apte à métaboliser un sucre en C5 avec une souche B présentant une efficacité applicative de référence.

Quel que soit la variante choisie, le procédé décrit ici peut comprendre au moins une étape de criblage permettant de sélectionner une souche satisfaisant au moins l'un des tests choisis parmi le test de croissance sur xylose, le test de croissance sur milieu CP et le test de séchage ou présentant une efficacité applicative EA au moins égale à 80%, de préférence au moins égale à 95%, et plus préférentiellement encore au moins égale à 105% de l'efficacité applicative de référence de la souche B.

L'étape de criblage peut être réalisée comme dernière étape du procédé.

Selon l'invention l'aptitude à métaboliser un sucre en C5 se traduit par l'aptitude à produire de l'éthanol à partir d'un milieu comprenant un sucre en C5 en condition anaérobie. Les souches aptes à métaboliser un sucre en C5 sont, de manière générale, des souches modifiées génétiquement dans lesquelles chaque gène de la voie des pentoses a été dérégulé, les copies du gène *GRE3* codant une aldose réductase sont délétées, le gène natif *XKS1* codant la xylulokinase a été surexprimé et qui ont subi une évolution dirigée.

Une levure apte à métaboliser un sucre en C5 est une souche de levure comportant au moins une copie d'un gène exogène codant pour une xylose isomérase, et au moins une copie d'un gène exogène codant pour une xylitol déshydrogénase de préférence issu de *Pichia stipitis.*

La xylose isomérase est un gène issu de *Clostridium, Piromyces, Bactéroïdes, Streptomyces, Haemophilus, Burkholderia, Enterococcus, Thermotoga, Fusobacterium, Geobacillus, Arthrobacter, Ciona, Physcomitrella, Cellvibrio, Chitinophaga, Saccharopolyspora* ou *Salinibacter,* et de préférence est un gène issu de *Clostridium phytofermentans* ou de *Piromyces sp.*

De plus, les souches aptes à métaboliser un sucre en C5 comportent les modifications suivantes :
- au moins une copie, de préférence au moins deux copies, d'un gène codant pour une aldose réductase, de préférence le gène *GRE3*, est délété, et
- le gène endogène codant pour une xylulokinase, de préférence le gène *XKS1*, est placé sous le contrôle d'un promoteur d'un gène non réprimé par l'anaérobiose ou par la répression catabolique induite par une quelconque source de carbone, et fortement exprimé lors de la fermentation alcoolique,
- au moins un gène endogène de la partie non oxydative de la voie des pentoses phosphates, de préférence choisi parmi les gènes *RPE1*, *RKI1*, *TKL1* et *TAL1*, et de manière particulièrement préférée la totalité de ces gènes est placée sous le contrôle d'un promoteur d'un gène non réprimé par l'anaérobiose ou par la répression catabolique induite par une quelconque source de carbone, et fortement exprimé lors de la fermentation alcoolique.
Les procédés de modifications génétiques peuvent être un de ceux décrits dans WO 2011/128552 ou dans WO2012/72793.

Selon un mode de réalisation du procédé d'hybridation décrit ici, ledit procédé comprend les étapes suivantes :
- sélection d'une souche (A) apte à métaboliser un sucre en C5;
- sélection d'une souche (B) ayant des performances compatibles avec une application industrielle;
- hybridation de ségrégeants de la souche (A) avec des ségrégeants de la souche (B).

Selon une variante, ledit procédé d'hybridation comprend les étapes supplémentaires suivantes :
- sélection d'une souche (A) apte à métaboliser un sucre en C5;
- sporulation de ladite souche (A) pour obtenir un ségrégeant X;
- sélection d'une souche (B) ayant des performances compatibles avec une application industrielle;
- sporulation de ladite souche (B) pour obtenir un ségrégeant Y;
- hybridation de X et Y.

La souche A est choisie parmi des souches modifiées génétiquement dans lesquelles chaque gène de la voie des pentoses a été dérégulé, les copies du gène codant les aldoses réductases sont délétées et qui ont subi une évolution dirigée.

Selon une forme de la description une souche A apte à métaboliser un sucre en C5 est une souche comportant au moins une copie du gène de *Pichia stipitis* XR codant pour l'enzyme xylose réductase, et au moins une copie du gène de *Pichia stipitis* XDH codant pour l'enzyme xylitol déshydrogénase

Selon une autre forme de la description une souche A apte à métaboliser un sucre en C5 est une souche de levure comportant au moins une copie d'un gène exogène codant pour une xylose isomérase, et au moins une copie d'un gène exogène codant pour une xylitol déshydrogénase de préférence issu de *Pichia stipitis.*

La xylose isomérase est un gène issu de *Clostridium, Piromyces, Bactéroïdes, Streptomyces, Haemophilus, Burkholderia, Enterococcus, Thermotoga, Fusobacterium, Geobacillus, Arthrobacter, Ciona, Physcomitrella, Cellvibrio, Chitinophaga, Saccharopolyspora* ou *Salinibacter,* et de préférence est un gène issu de *Clostridium phytofermentans* ou de *Piromyces sp.*

De plus, les souches A aptes à métaboliser un sucre en C5 comportent les modifications suivantes :
- au moins une copie, de préférence au moins deux copies, d'un gène codant pour une aldose réductase, de préférence le gène *GRE3*, est délété, et
- le gène endogène codant pour une xylulokinase, de préférence le gène *XKS1*, est placé sous le contrôle d'un promoteur d'un gène non réprimé par l'anaérobiose ou par la répression catabolique induite par une quelconque source de carbone, et fortement exprimé lors de la fermentation alcoolique,
- au moins un gène endogène de la partie non oxydative de la voie des pentoses phosphates, de préférence choisi parmi les gènes *RPE1*, *RKI1*, *TKL1* et *TAL1*, et de manière particulièrement préférée la totalité de ces gènes, sont placés sous le contrôle d'un promoteur d'un gène non réprimé par l'anaérobiose ou par la répression catabolique induite par une quelconque source de carbone, et fortement exprimé lors de la fermentation alcoolique.

Les procédés de modifications génétiques peuvent être un de ceux décrits dans WO 2011/128552 ou dans WO2012/72793.

La souche A peut également être choisie par un procédé de criblage de souche. Seules les souches satisfaisant le test de croissance sur xylose et/ou le test de croissance sur milieu CP, tels que définis précédemment sont sélectionnées.

La souche B peut également être choisie par un procédé de criblage de souche. Seules les souches présentant une efficacité applicative EA au moins égale à 80%, de préférence au moins égale à 95% et plus préférentiellement encore au moins égale à 105% de l'efficacité applicative de référence seront sélectionnées.

Lorsque la souche est une souche panaire, la souche (B) qui sera choisie présentera une force fermentative supérieure à 5 mL.2h⁻¹. g de farine⁻¹ de préférence supérieure à 6 mL.2h⁻¹. g de farine⁻¹ et plus préférentiellement encore supérieure à 8 mL.2h⁻¹. g de farine⁻¹.

Le procédé mettant en oeuvre des étapes d'obtention de ségrégeants peut également comprendre une étape de criblage du ségrégeant X et/ou une étape de criblage du ségrégeant Y. Seuls les ségrégeants X satisfaisant le test de croissance sur xylose et/ou le test de croissance sur milieu CP, tels que définis précédemment sont sélectionnés. Seuls les ségrégeants Y présentant une efficacité applicative EA au moins égale à 80%, de préférence au moins égale à 95% et plus préférentiellement encore au moins égale à 105% de l'efficacité applicative de référence seront sélectionnés.

De manière préférentielle le ségrégeant X est choisi parmi les souches déposées à la Collection Nationale des Cultures de Microorganismes (CNCM, Institut Pasteur, 28 rue du Docteur Roux, 75724 Paris cedex 15) le 23 août 2012 sous le N° I-4672 et I-4673 et le ségrégeant Y est la souche déposée à la Collection Nationale des Cultures de Microorganismes (CNCM, Institut Pasteur, 28 rue du Docteur Roux, 75724 Paris cedex 15) le 23 août 2012 sous le N° I-4669.

L'invention a aussi pour objet un procédé de production de levures en « Fed Batch » aérobie sur un substrat comprenant au moins un sucre C5 à partir d'une souche telle que décrite précédemment. Par procédé « Fed Batch » aérobie, on entend une « culture en mode semi-continu » ou « culture semi-continue » ou « mode semi-continu », désigne ici une culture dans un fermenteur (ou réacteur) qui est alimenté progressivement par le milieu de culture, mais dont aucun volume de milieu n'est soutiré. Dans un tel procédé, le volume de culture est variable (et généralement croissant) dans le fermenteur, le débit d'alimentation pouvant être constant ou variable.

Le procédé « Fed Batch » est généralement réalisé dans les conditions décrites dans le livre de référence « Yeast Technology », Chapitre 6, 2ème édition, 1991, G. Reed et T.W. Nagodawithana, publié par Van Nostrand Reinhold, ISBN 0-442-31892-8.

Selon l'invention le taux de multiplication est généralement de 1,16. De manière générale, le taux d'utilisation des sucres en C5 par les souches de l'invention dépend de la nature de ces souches et peut aller jusqu'à 100% dans certaines conditions. Ainsi une souche destinée à une application finale en panification peut, lors de sa multiplication, utiliser jusqu'à 50% des sucres en C5 compris dans le substrat de fermentation, alors qu'une souche destinée à une application en production d'éthanol peut utiliser jusqu'à 100% des sucres en C5.

Un autre objet de l'invention est l'utilisation des levures obtenues dans au moins une application industrielle choisie parmi la panification, la production de biomasse, la production d'arômes, la production de métabolites secondaires, la production de protéine, la production d'ARN, la production d'éthanol, la brasserie ou la production d'extrait de levure.
Les exemples suivants illustrent l'invention sans en limiter la portée.

### Exemples :

### Exemple 1 : Préparation de ségrégeants X

Afin d'obtenir des ségrégeants de la souche CNCM I-4538, cette dernière a été cultivée pendant 24 heures dans du milieu YPG contenant 10 g/kg d'Extrait de Levure (EXL) type J100, 10 g/kg de peptone et 20 g/kg de glucose. La suspension de cellules obtenue a ensuite été transférée sur une boite de Pétri contenant un milieu SAA constitué de 20 g/kg d'agarose et de 7,5 g/kg d'acétate de sodium. Après 5 jours d'incubation à 30°C, les tétrades sont disséquées à l'aide d'un micromanipulateur. Les boites ainsi préparées sont incubées 48 heures à 30°C.

L'une des limites à l'utilisation des ségrégeants obtenus concerne leur signe sexuel. En effet, lors de la construction par biologie moléculaire des souches capables de fermenter le xylose, certains gènes essentiels à cette voie ont été introduits dans un locus génétiquement lié à Mat alpha. Ce point implique donc que tous les ségrégeants issus de la souche déposée à la Collection Nationale des Cultures de Microorganismes (CNCM, Institut Pasteur, 28 rue du Docteur Roux, 75724 Paris cedex 15) le 5 octobre 2011 sous la référence CNCM I-4538 et qui sont capables de métaboliser le xylose sont de signe Mat alpha. Afin de lever cette limitation, le signe sexuel de ces ségrégeants a été changé. Pour cela, nous avons utilisé un processus qui existe dans la nature. En effet, certaines levures sauvages, lorsqu'elles sont à l'état haploïde, sont capables de changer de signe sexuel. Ce processus se fait sous l'action de la recombinase HO. Cette dernière n'est pas fonctionnelle dans la souche déposée à la CNCM sous la référence CNCM I-4538 et par voie de conséquence, elle ne fonctionne pas non plus dans cette souche. Nous avons introduit un plasmide réplicatif, qui contient un gène codant une recombinase HO fonctionnelle, dans les ségrégeants d'intérêt. Cette méthode s'appuie sur les travaux de Herskowitz et Jensen publiés en 1991. Le gène HO porté par le plasmide en question a été placé sous la dépendance d'un promoteur induit par le galactose. Les ségrégeants ainsi transformés ont été incubés pendant 16 heures dans un milieu contenant 10 g/ kg d'EXL type J100, 10 g/ kg de Peptone et 20 g/ kg de galactose. Les cellules ont ensuite été étalées sur un milieu solide contenant 10 g/kg d'EXL type J100, 20 g/kg d'agarose et 20 g/kg de glucose. Après avoir obtenu des clones, le changement de signe sexuel a été vérifié en réalisant une PCR sur le locus d'expression du signe sexuel Cette réaction est réalisée en utilisant comme matrice l'ADN génomique de chaque ségrégeant potentiel. Les oligonucléotides sont d'une part une amorce propre au locus MAT exprimé sur le chromosome III de la levure (amorce Mat1 : AGTCACATCAAGATCGTTTATGG) et deux autres amorces l'une propre à Mat alpha (amorce Mat2 : GCACGGAATATGGGACTACTTCG) et l'autre propre à Mat a (amorce Mat3 : ACTCCACTTCAAGTAAGAGTTTG).

Le génotype Mat a des levures se caractérise par la présence d'un amplicon de 544 paires de bases détectable sur gel d'agarose à 0,8 %.

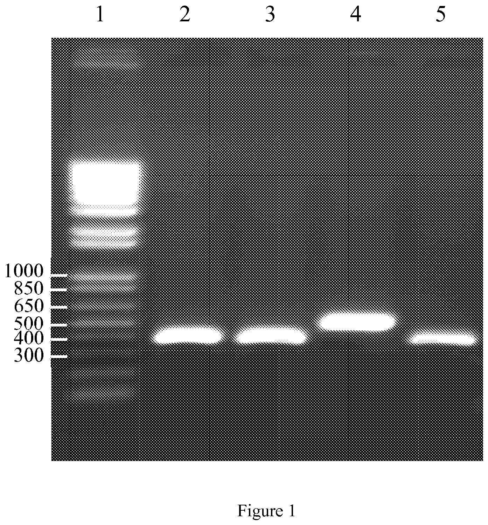

Les résultats obtenus sont présentés sur la figure 1.

### Exemple 2 : Préparation de ségrégeants Y et vérification de la capacité des ségrégeants à transmettre une force fermentative élevée après hybridation.

Préparation du ségrégeant : le ségrégeant déposé à la CNCM sous le N° I-4669 a été préparé à partir de la souche panaire déposée à la CNCM sous le N° I-2970. Le mode de préparation suivi est identique à celui décrit à l'exemple 1.

Vérification de la transmission : des hybrides 4539 et 4550 ont été préparés par hybridation des ségrégeants issus des souches panaires déposées à la NCYC sous le N° 995 et à la CNCM sous le N° I-2970.

Les forces fermentatives sur Pâte Normale (PN) et Pâte Sucrée à 2 g (PS 2g) indiquées dans le tableau suivant montre que le ségrégeant I-4669 peut transmettre une force fermentative élevée après hybridation.

**Tableau 1**

| Souches | I-2970 | NCYC-995 | 4539 | 4550 |
|---|---|---|---|---|
| Force sur PN | 161 | 146 | 187 | 167 |
| Force sur PS 2g | 139 | 117 | 155 | 148 |

### Exemple 3 : hybridation

Les différents ségrégeants préparés ont été croisés comme indiqué dans le tableau ci-après :

**Tableau 2 : Croisements réalisés**

| | | Mat alpha | | | | |
|---|---|---|---|---|---|---|
| | | X10S3 | X10S2 | X10S1 | PS27 | Ps28 |
| | | | CNCM **N°I-4673** | CNCM N° **I-4672** | | |
| Mat a | X10S4 | | | | HC5-G | HC5-H |
| | *X10S5* | | | | HC5-I | HC5-J |
| | X10S6 | | | | HC5-K | HC5-L |
| | PS316 | HC5-A | HC5-C | HC5-B | | |
| | **I-4669** | | **I-4671** | **I-4670** | | |
| | P3S8 | HC5-D | HC5-F | HC5-E | | |

### Exemple 4 : Criblage

Les souches hybrides obtenues à l'exemple 3 ci-dessus ont été soumis aux différents criblages suivants:

### 1/ Test de croissance sur xylose

Ce premier test concerne la capacité à se multiplier en utilisant le xylose comme source de carbone.

Afin de déterminer la capacité des souches à se multiplier en utilisant le xylose comme source de carbone, nous les avons inoculées à hauteur de 2x106 cellules/mL dans 50 mL de milieu YFX dont la composition est donnée ci-dessus et maintenues à 30°C sous agitation de 150 rpm.

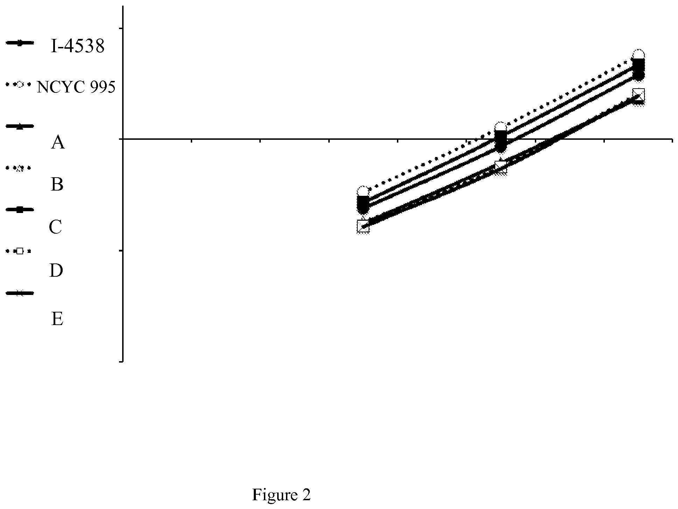

L'évolution de la biomasse est déterminée par un suivi de la turbidité mesurée au spectrophotomètre à une longueur d'onde de 600 nm. Le résultat de ce suivi est représenté sur la figure 2.

L'évolution de la biomasse sur le milieu YFX nous a permis de sélectionner 4 souches. Il s'agit des hybrides HC5-A, HC5-B, HC5-C et HC5-D qui présentent une bonne capacité à utiliser le xylose comme source de carbone. De plus, ces souches sont capables de prendre rapidement en charge le xylose, là où les hybrides HC5-K et HC5-L n'ont pas été retenus en raison de leur retard pour initier leur croissance sur xylose.

### 2/ Test de croissance sur milieu CP

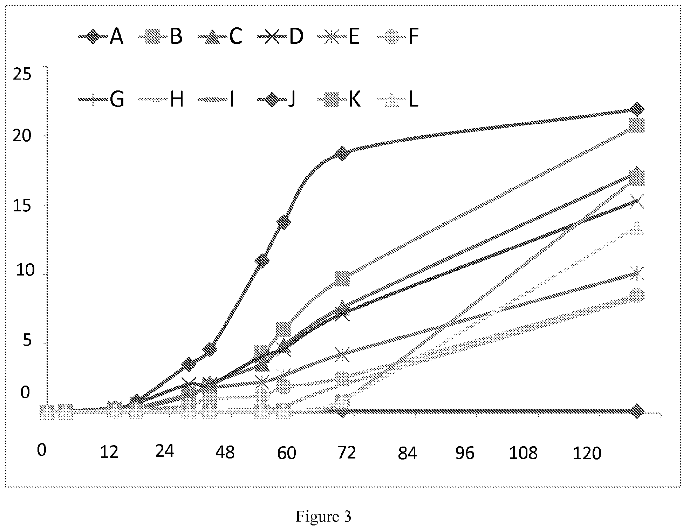

Un autre critère important est la vitesse de multiplication maximale des cellules. Afin de la déterminer, les levures ont été inoculées dans 50 ml de milieu CP à hauteur de 2 x 10⁶ cellules par mL. Ce milieu est très riche puisqu'il contient 10 g/kg d'EXL et 10 g/kg de bactopeptone ainsi que 100 g/kg de saccharose. L'évolution de la turbidité est mesurée par spectrophotométrie à 600 nm. Il s'agit ensuite d'analyser la vitesse de multiplication maximale en identifiant la période durant laquelle la biomasse croît de manière exponentielle. La figure 3 représente l'évolution de la turbidité selon une échelle logarithmique et en fonction du temps de culture.

Dans l'espace compris entre 3,5 et 7,5 heures, l'évolution de la turbidité est exponentielle, ce qui est confirmé par l'aspect linéaire sur notre graphique qui utilise une échelle logarithmique. La vitesse de multiplication est définie comme étant la dérivée de l'évolution de la turbidité à 600 nm en fonction du temps. Or, comme les différentes droites semblent parallèles, cela suggère que les vitesses de multiplication semblent comparables à celles de la souche déposé à la NCYC sous la référence NCYC 995 et de la souche déposée à la CNCM sous la référence CNCM I-4538 sur ce milieu.

### 3/ Test de la force fermentative après un pseudo fedbatch

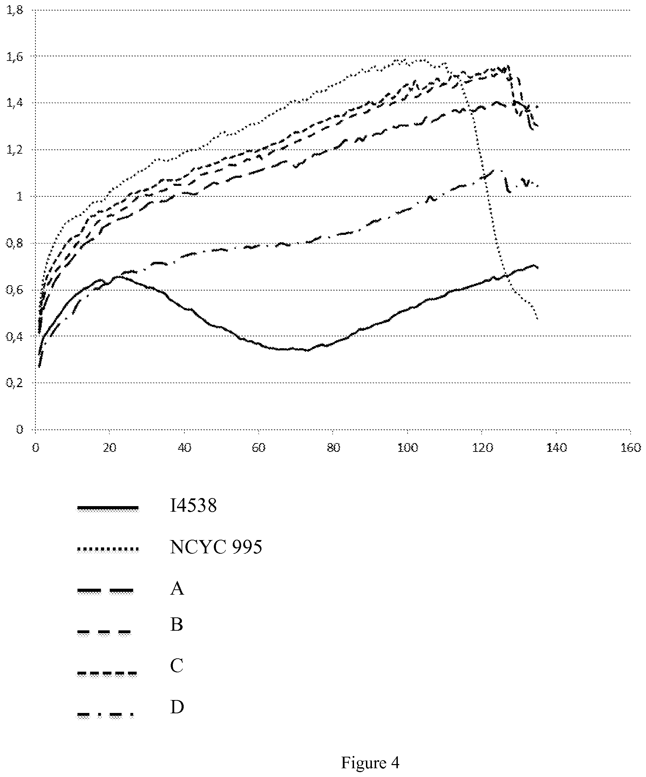

Les Hybrides HC5-B et HC5-C (souches I-4670 et I-4671) ont été propagés en utilisant du raffinose comme source de carbone. L'utilisation de ce tri holoside permet de délivrer le sucre progressivement à la levure. En effet, le raffinose est hydrolysé lentement par l'invertase des levures. Après avoir standardisé les quantités de biomasse des différentes production en mesurant la quantité de matière sèche levure par unité de volume à l'aide d'un appareil SMART System5™ (CEM corporation USA), nous avons analysé le dégagement gazeux à l'aide d'un fermentomètre risographe. L'évolution des dégagements gazeux pour un test sur pâte normale, représentée sur la figure 4 est réalisée comme suit :
- Une suspension de levure est préparée de la façon suivante : la quantité équivalente à 1 g de matière sèche de la levure à tester est mise en suspension et complétée à 100ml de solution à 27g/L de NaCl et 4g/L de (NH4)2SO4.
- 15ml de la suspension ci-dessus (soit 150 mg de matière sèche levure) sont équilibrés à une température de 30°C pendant 15 minutes.
- A cette suspension sont ajoutés 20g de farine préalablement équilibrés une nuit à 30°C. L'ensemble est homogénéisé pendant une durée de 35 secondes.
- La pâte formée est incubée dans un récipient fermé hermétiquement placé à 30°C. Le dégagement gazeux (exprimé en ml sous 760 mm Hg) est enregistré sur une durée totale de 120 minutes.

Le protocole de mesure du dégagement gazeux pour un test en Pâte Sucrée (PS) est identique à celui suivi pour PN sauf que du sucre (ici 2g) est ajouté avec les autres ingrédients secs.

Les profils obtenus mettent en évidence que la répression catabolique est importante dans la souche déposée à la CNCM sous la référence CNCM I-4538, ce qui induit un phénomène de diauxie important dit « creux maltose », ce qui la disqualifie pour les applications panaires. En revanche, il est à noter que les hybrides HC5-A, B, C et D, bien que plus lents que la souche déposée à la NCYC sous le N° NCYC 995, semblent présenter des profils intéressants. Les Hybrides HC5-B et HC5-C (souches I-4670 et 4671) donnent les meilleurs résultats.

### Exemple 5 :

Chacune des souches déposées sous le n°I-4670 et n°I-4671, a été soumise à un test de croissance sur xylose, à un test de croissance sur milieu CP et leur force fermentative a été mesurée.
Les résultats obtenus sont présentés dans le tableau ci-dessous:

| Souche | CNCM n°I-4670 | CNCM n°I-4671 |
|---|---|---|
| Croissance sur xylose (vitesse de multiplication) | 3,5 heures / génération | 6 heures / génération |
| Croissance du milieu CP (vitesse de multiplication) | 0.71 | 0.66 |
| Force fermentative | 139 | 143 |

### Exemple 6 :

Chacune des souches déposées sous le n°I-4670 et n°I-4671 a été multipliée selon un procédé Fed batch aérobie pendant 18 heures. Les forces fermentatives des levures obtenues après séchage ont été mesurées selon un test Pâte Normale (PN) et selon un test Pâte Sucrée 2 grammes (PS2g). Les résultats obtenus sont reproduits ci-après :

| | EA de la souche référencée NCYC 995 | I-4670 | I-4671 |
|---|---|---|---|
| milieu de culture | 100% C6 | C5/C6 de 0,42 | C5/C6 de 0,42 |
| Rendement de production | 79,2% | 65,7% | 66,0% |
| Force fermentative PN | 118 | 119 | 120 |
| Force fermentative PS2g | 114 | 116 | 107 |

## Revendications

1. Souche de levure du genre saccharomyces apte à se multiplier en condition aérobie sur un substrat comprenant au moins un sucre en C5, **caractérisée en ce qu'**elle satisfait un test de croissance sur xylose ou qu'elle satisfait un test de croissance sur milieu CP et **en ce que** dans son génome les gènes RPE1, RKI1, TKL1 et TAL1 de la voie des pentoses ont été dérégulés, les copies du gène GRE 3 codant une aldose réductase sont délétées, le gène natif XKS1 a été surexprimé, au moins une copie d'un gène de *Pichia Stipitis* codant pour une xylose déshydrogénase a été introduite et au moins une copie d'un gène codant pour une xylose isomérase a été introduite.
dans laquelle le test de croissance sur milieu CP consiste à suivre au cours du temps par spectrophotométrie à une longueur d'onde de 600 nm l'évolution de la turbidité d'une solution contenant 50 mL d'un milieu de culture CP qui contient 10 g/kg d'EXL type J100 et 10 g/kg de bactopeptone ainsi que 100 g/kg de saccharose, ledit milieu étant inoculé par 10⁸ cellules à tester à 30°C sous agitation constante à 150 rpm, et dans laquelle ledit test de croissance sur milieu CP est satisfait lorsque l'évolution de la densité optique est exponentielle entre 3,5 et 7,5 heures.

2. Souche selon la revendication 1 **caractérisée en ce que** le gène codant pour la xylose isomérase est issu de bactéries du genre *Clostridium* et de préférence de *Clostridium phytofermentans.*

3. Souche selon l'une des revendications précédentes **caractérisée en ce qu'**elle satisfait un test de croissance sur xylose et qu'elle satisfait un test de croissance sur milieu CP.

4. Souche selon l'une des revendications précédentes choisie dans le genre Saccharomyces et de préférence *Saccharomyces cerevisiae.*

5. Souche selon l'une quelconque des revendications 1 à 4, **caractérisée en ce qu'**elle satisfait un test de séchage.

6. Souche selon l'une quelconque des revendications 1 à 5, **caractérisée en ce qu'**elle présente une efficacité applicative EA au moins égale à 80%, de préférence au moins égale à 95% et plus préférentiellement encore au moins égale à 105% de l'efficacité applicative de référence.

7. Souche selon la revendication 6, **caractérisée en ce que** l'efficacité applicative est choisie dans le groupe comprenant la force fermentative, la viabilité, le rendement de production de biomasse, d'arôme, de métabolite secondaire, de protéine, d'ARN ou d'éthanol.

8. Souche de levure selon l'une quelconque des revendications 1 à 7, qui est une souche de levure panaire.

9. Souche selon la revendication 8, dont l'efficacité applicative est la force fermentative et est au moins égale à 95% de la force fermentative de référence qui est de 100 mL de CO₂ en 2 heures à partir de 20g de farine.

10. Souche de levure selon la revendication 9 déposée à la CNCM le 23 août 2012 sous le N°I-4670.

11. Souche de levure selon la revendication 9 déposée à la CNCM le 23 août 2012 sous le N° I-4671.

12. Utilisation des levures obtenues par culture des souches telles que définies selon l'une quelconque des revendications 1 à 11 dans une application industrielle choisie parmi la panification, la production de biomasse, la production d'arômes, la production de métabolites secondaires, la production de protéine, la production d'ARN, la production d'éthanol, la brasserie ou la production d'extrait de levure.

13. Procédé de production de levures en « Fed Batch » aérobie sur un substrat comprenant au moins un sucre C5 à partir d'une souche selon l'une des revendications 1 à 11.

14. Utilisation de levures produites selon le procédé de la revendication 13, dans une application industrielle choisie parmi la panification, la production de biomasse, la production d'arômes, la production de métabolites secondaires, la production de protéine, la production d'ARN, la production d'éthanol, la brasserie, l'oenologie, ou la production d'extrait de levure.

15. Souche selon l'une quelconque des revendications 1 à 11 ou procédé la revendication 13, **caractérisé en ce que** ledit substrat en C5 comprend au moins un sucre C6.

16. Souche ou procédé selon la revendication 15, **caractérisé en ce que** le rapport des sucres C5/C6 est compris entre 0,1 et entre 2.

17. Souche ou procédé selon la revendication 15 ou la revendication 16, **caractérisé en ce que** le sucre C5 est du xylose.

18. Souche ou procédé selon l'une quelconque des revendications 15 à17, **caractérisé en ce que** les C6 sont choisis parmi le glucose, le fructose, le galactose, le mannose ou sont issus de la dégradation de diholoside comme le saccharose, le maltose le tréhalose ou l'isomaltose.

## Patentansprüche

1. Hefestamm der Gattung *Saccharomyces*, der sich unter aeroben Bedingungen auf einem Substrat mit mindestens einem C5-Zucker vervielfachen kann, **dadurch gekennzeichnet, dass** er einen Wachstumstest auf Xylose erfüllt oder dass er einen Wachstumstest auf CP-Medium erfüllt und **dadurch gekennzeichnet, dass** in seinem Genom die Gene RPE1, RK11, TKL1 und TAL1 des Pentosewegs dereguliert sind, die Kopien des Gens GRE 3, das eine Aldosereduktase kodiert, gelöscht sind, das native Gen XKS1 überexprimiert ist, mindestens eine Kopie eines Gens von *Pichia Stipitis*, das eine Xylose-Dehydrogenase kodiert, eingeführt ist und mindestens eine Kopie eines Gens, das eine Xylose-Isomerase kodiert, eingeführt ist,
bei dem der Wachstumstest auf CP-Medium darin besteht, dass mittels Spektrophotometrie bei einer Wellenlänge von 600 nm der zeitliche Verlauf der Entwicklung der Trübung einer Lösung verfolgt wird, die 50 ml eines CP-Kulturmediums enthält, das 10 g/kg EXL Typ J100 und 10g/kg Bactopepton sowie 100 g/kg Saccharose enthält,
wobei das Medium mit 10⁸ zu testenden Zellen bei 30°C unter ständigem Rühren bei 150 U/min inokuliert wurde, und wobei der Wachstumstest auf dem CP-Medium erfüllt ist, wenn die Entwicklung der optischen Dichte zwischen 3,5 und 7,5 Stunden exponentiell verläuft.

2. Stamm gemäß Anspruch 1, **dadurch gekennzeichnet, dass** das Gen, das die Xylose-Isomerase kodiert, aus Bakterien der Gattung *Clostridium* und vorzugsweise *Clostridium Phytofermentans* stammt.

3. Stamm gemäß einem der vorhergehenden Ansprüche, **dadurch gekennzeichnet, dass** er einem Wachstumstest auf Xylose erfüllt und dass er einem Wachstumstest auf CP-Medium erfüllt.

4. Stamm gemäß einem der vorhergehenden Ansprüche, ausgewählt aus der Gattung *Saccharomyces* und vorzugsweise *Saccharomyces cerevisiae.*

5. Stamm gemäß einem der Ansprüche 1 bis 4, **dadurch gekennzeichnet, dass** er einem Trocknungstest erfüllt.

6. Stamm gemäß einem der Ansprüche 1 bis 5, **dadurch gekennzeichnet, dass** er eine Anwendungseffizienz EA von mindestens 80%, vorzugsweise mindestens 95% und besonders bevorzugt mindestens 105% der Referenzanwendungseffizienz aufweist.

7. Stamm gemäß Anspruch 6, **dadurch gekennzeichnet, dass** die Anwendungseffizienz ausgewählt ist aus der Gruppe bestehend aus Fermentationskraft, Lebensfähigkeit, Ausbeute der Herstellung von Biomasse, von Aroma, von Sekundärmetabolit, von Protein, von RNA oder von Ethanol.

8. Stamm gemäß einem der Ansprüche 1 bis 7, der ein Triebmittelhefestamm ist.

9. Stamm gemäß Anspruch 8, bei dem die Anwendungseffizienz die Fermentationskraft ist und mindestens 95% der Referenzfermentationskraft entspricht, die 100 ml CO₂ in 2 Stunden aus 20 g Mehl beträgt.

10. Hefestamm gemäß Anspruch 9, der am 23. August 2012 bei der CNCM unter der Nummer N°1-4670 eingereicht wurde.

11. Hefestamm gemäß Anspruch 9, der am 23. August 2012 bei der CNCM unter der Nummer N°1-4671 eingereicht wurde.

12. Verwendung der Hefen, die durch Kultivieren der Stämme gemäß einem der Ansprüche 1 bis 11 erhalten werden, in einer industriellen Anwendung, ausgewählt aus Brotbacken, Biomasseherstellung, Aromaherstellung, Herstellung von Sekundärmetaboliten, Proteinherstellung, RNA-Herstellung, Ethanolherstellung, Brauen oder Hefeextraktherstellung.

13. Verfahren zur Herstellung von aeroben "Fed-Batch"-Hefen auf einem Substrat, das mindestens einen C5-Zucker umfasst, aus einem Stamm gemäß einem der Ansprüche 1 bis 11.

14. Verwendung von gemäß dem Verfahren von Anspruch 13 hergestellten Hefen in einer industriellen Anwendung, ausgewählt aus Brotbacken, Biomasseherstellung, Aromaherstellung, Herstellung von Sekundärmetaboliten, Proteinherstellung, RNA-Herstellung, Ethanolherstellung, Brauen, Önologie oder Hefeextraktherstellung.

15. Stamm gemäß einem der Ansprüche 1 bis 11 oder Verfahren gemäß Anspruch 13, **dadurch gekennzeichnet, dass** das C5-Substrat mindestens einen C6-Zucker umfasst.

16. Stamm oder Verfahren gemäß Anspruch 15, **dadurch gekennzeichnet, dass** das Verhältnis der C5/C6-Zucker zwischen 0,1 und 2 liegt.

17. Stamm oder Verfahren gemäß Anspruch 15 oder 16, **dadurch gekennzeichnet, dass** der C5-Zucker Xylose ist.

18. Stamm oder Verfahren gemäß einem der Ansprüche 15 bis 17, **dadurch gekennzeichnet, dass** die C6 ausgewählt sind aus Glukose, Fruktose, Galaktose, Mannose oder aus dem Abbau von Disacchariden, wie Saccharose, Maltose, Trehalose oder Isomaltose stammen.

## Claims

1. Yeast strain of the saccharomyces genus capable of multiplying under aerobic conditions on a substrate comprising at least one C5 sugar, **characterised in that** it satisfies a growth test on xylose or that it satisfies a growth test on CP medium and **in that** in its genome, the genes RPE1, RKI1, TKL1 and TAL1 of the pentose pathway have been deregulated, the copies of the GRE3 gene coding for an aldose reductase are deleted, the native XKS1 gene has been overexpressed, at least one copy of a *Pichia Stipitis* gene coding for a xylose dehydrogenase has been introduced and at least one copy of a gene coding for a xylose isomerase has been introduced.
wherein the growth test on CP medium consists in monitoring over time by spectrophotometry at a wavelength of 600nm, the variation in turbidity of a solution containing 50mL of a CP culture medium which contains 10g/kg of EXL type J100 and 10g/kg bactopeptone, as well as 100g/kg of sucrose, said medium being inoculated with 10⁸ cells to be tested at 30°C with constant stirring at 150rpm, and wherein said growth test on CP medium is satisfied when the variation in the optical density is exponential between 3.5 and 7.5 hours.

2. Strain according to claim 1, **characterised in that** the gene coding for the xylose isomerase originates from bacteria of the genus *Clostridium* and preferably *Clostridium phytofermentans.*

3. Strain according to one of the preceding claims, **characterised in that** it satisfies a growth test on xylose and that it satisfies a growth test on CP medium.

4. Strain according to one of the preceding claims, selected from the genus Saccharomyces and preferably *Saccharomyces cerevisiae.*

5. Strain according to any one of claims 1 to 4, **characterised in that** it satisfies a drying test.

6. Strain according to any one of claims 1 to 5, **characterised in that** it has an application efficiency EA at least equal to 80%, preferably at least equal to 95% and even more preferably at least equal to 105% of the reference application efficiency.

7. Strain according to claim 6, **characterised in that** the application efficiency is selected from the group consisting of the fermentative power, the viability, the yield of biomass, flavour, secondary metabolite, protein, RNA or ethanol production.

8. Yeast strain according to any one of claims 1 to 7, which is a breadmaking yeast strain.

9. Strain according to claim 8, which application efficiency is the fermentative power and is at least equal to 95% of the reference fermentative power which is 100mL of CO₂ in 2 hours from 20g of flour.

10. Yeast strain according to claim 9, deposited at the CNCM on 23 August 2012 under the N°I-4670.

11. Yeast strain according to claim 9, deposited at the CNCM on 23 August 2012 under the N°I-4671.

12. Use of the yeasts obtained by culturing strains as defined according to any one of claims 1 to 11 in an industrial application chosen from breadmaking, biomass production, flavour production, the production of secondary metabolites, protein production, RNA production, ethanol production, brewing or the production of yeast extract.

13. Method for aerobic "Fed Batch" production of yeast on a substrate comprising at least one C5 sugar from a strain according to any one of claims 1 to 11.

14. Use of yeast produced according to the method of claim 13, in an industrial application selected from breadmaking, biomass production, flavour production, the production of secondary metabolites, protein production, RNA production, ethanol production, brewing, winemaking, or the production of yeast extract.

15. Strain according to any one of claims 1 to 11 or method according to claim 13, **characterised in that** said C5 substrate comprises at least one C6 sugar.

16. Strain or method according to claim 15, **characterised in that** the ratio of the C5/C6 sugars is between 0.1 and between 2.

17. Strain or method according to claim 15 or claim 16, **characterised in that** the C5 sugar is xylose.

18. Strain or method according to any one of claims 15 to 17, **characterised in that** the C6 are selected from glucose, fructose, galactose, mannose or originate from the degradation of disaccharide such as sucrose, maltose trehalose or isomaltose.
